# EUROPEAN PATENT SPECIFICATION

(11) **EP 3 243 383 B1**
(45) Date of publication and mention of the grant of the patent: **03.06.2020**
(21) Application number: 16768423.2
(22) Date of filing: 09.03.2016
(51) Int. Cl.: A01N 1/02

(54) **FREEZING BAG CONTAINER**
GEFRIERBEUTELBEHÄLTER
RÉCEPTACLE DE SAC DE CONGÉLATION

(30) Priority: 24.03.2015 JP 2015061285
(43) Date of publication of application: 15.11.2017
(73) Proprietor: Terumo Kabushiki Kaisha, Tokyo 151-0072 (JP)
(72) Inventor: MATSUMURA, Masaki, Ashigarakami-gun Kanagawa 259-0151 (JP)
(74) Representative: Prüfer & Partner mbB Patentanwälte · Rechtsanwälte
(86) International application number: PCT/JP2016/057339
(87) International publication number: WO 2016/152520

(56) References cited:
- WO-A1-2014/099515
- WO-A2-2009/086136
- JP-A- 2002 095 708
- JP-A- 2004 121 669
- JP-A- 2014 124 234
- JP-U- H0 726 375
- JP-U- S51 106 616
- JP-U- S59 170 163
- US-A- 3 952 536
- US-A1- 2014 144 800
- US-A1- 2015 068 232

## Description

### Technical Field

The present invention relates to a freezing bag container.

### Background Art

In the case of preserving biological tissue such as cells for a long time, the biological tissue is filled into a freezing bag, and is cryopreserved in the state in which the freezing bag filled with the biological tissue is immersed in liquid nitrogen. The freezing bag includes a bag main body which is formed in a bag shape by fusing a plastic film or films and which is equipped with a filling port and a discharge port. The biological tissue is suspended in a cryopreservation solution or the like, the suspension is filled into the bag main body through the filling port, and the biological tissue is cryopreserved. At the time of using the biological tissue, the freezing bag preserved in liquid nitrogen is taken out, is put to thawing, and the biological tissue is taken out via the discharge port, to be used (JP 2003-205016 A).

If biological tissue is frozen by abruptly immersing it in liquid nitrogen, cells would be damaged. It is said, therefore, that it is preferable to freeze the biological tissue by cooling it down to -80°C at a rate of approximately 1°C/minute to 5°C/minute by use of a programmable freezer or the like (JP 2003-267471 A). On the other hand, at the time of thawing the frozen biological tissue, a method of warming the frozen biological tissue by immersing it in hot water at 37°C to achieve rapid thawing or the like method is generally adopted, for reducing damage to the cells (JP 2002-253206 A).

Since a freezing bag is produced using a fragile blank material such as plastic film, the freezing bag may be broken by mistake by the operator at the time of cooling, freezing, preservation, warming, thawing or the like. In addition, when freezing bags are immersed in liquid nitrogen as they are, the freezing bags are frozen in a mutually adhered state and, therefore, it is difficult to take the freezing bag out of liquid nitrogen. In order to solve such a problem, bags and containers for protecting the freezing bags have been developed.

For instance, JP 2003-267471 A and JP 2000-140069 A disclose a covering bag which is obtained by forming a film with excellent impact resistance into a bag shape and which is for protecting a freezing bag, and a metallic container for accommodating the covering bag covering the freezing bag. The covering bag and the metallic container are designed for consistently protecting and storing the freezing bag, from freezing to thawing of the cells in the freezing bag. However, at the time of freezing, part of the freezing bag may be rapidly cooled, and cells in the freezing bag may be frozen non-uniformly. At the time of thawing, on the other hand, the freezing bag may fail to be warmed up appropriately, and insufficient thawing of the biological tissue may occur, resulting in a lowered recovery rate.

US 3,952,536 discloses a restraining plate set, comprising two plates that are clipped together and restrain a fluid filled bag to be frozen, e.g. in liquid nitrogen. The flat planar surfaces of the bag contacting surfaces of the plates may be interrupted with ridges to lock or immobilise the slack filled bag in place for handling prior to freezing and to impart additional structural integrity to the frozen product after freezing.

### Summary of Invention

### Technical Problem

It is an object of the present invention to provide a container which can protect a freezing bag filled with biological tissue, consistently from freezing to thawing, can solve the above-mentioned problems, and can give an enhanced recovery rate through appropriate freezing and thawing.

### Technical Solution

The present inventor made extensive and intensive researches for solving the above-mentioned problems. In his/her researches, the inventor paid attention to the fact that for enhancing recovery rate of biological tissue put to freezing and thawing, it is necessary that excessively rapid freezing can be restrained at the time of freezing and that comparatively rapid warming can be achieved at the time of thawing. As a result of his further research, the present inventor has found out that by providing an air gap between a freezing bag and an inner surface of a container and providing an opening for securing a flow path of hot water, it is possible to provide a freezing bag container by which a series of operations of cooling, freezing, preservation, warming, thawing and the like can be consistently performed in a state wherein a freezing bag is contained and protected in the container and by which a good recovery rate is achieved. Based on the finding, the present invention provides a container in combination with a freezing bag as defined in claim 1. Preferred embodiments are defined in the dependent claims. The container is thermally conductive and substantially rectangular parallelepiped-shaped, for containing the freezing bag that in use is filled with biological tissue, and for cooling and warming the freezing bag, the container including:
at least one opening formed in a side surface of the container; and
at least two ridges formed at an inner surface of the container to form an air gap between the inner surface and the freezing bag.

The opening is provided in each of two opposed side surfaces of the container, to form a flow path between the opening on one side and the opening on the other side.

The ridges are formed at an inner surface or surfaces of an upper surface and/or a lower surface of a main body of the container in such a manner as to extend in parallel to a flow path direction of the flow path.

A section of the ridge may have an arcuate shape.

The opening may have such a shape as to be connectable to a pipe.

The container may further include protective bag for containing and protecting the freezing bag, wherein the protective bag has ridges formed with engaging grooves for engagement with the at least two ridges of the container.

The opening may have an openable and closable structure.

### Advantageous Effects

According to the present invention, a container is provided, by which cooling and heating of a freezing bag can be performed consistently and appropriately. Specifically, an air gap is provided between the freezing bag and the container, whereby at the time of freezing, thermal conductivity between the freezing bag and the container is lowered and rapid cooling of the freezing bag is prevented. Therefore, biological tissue in the freezing bag is frozen uniformly, so that recovery rate is enhanced. On the other hand, the container is provided with an opening, whereby hot water can be taken in appropriately. At the time of thawing, hot water flows into the air gap, whereby the area of contact between the hot water and the freezing bag is increased, and the freezing bag is warmed up appropriately. Therefore, re-freezing of the biological tissue in the freezing bag does not occur, so that recovery rate is enhanced. Especially, by the container in combination with the freezing bag in accordance with the present invention, a series of operations of cooling, freezing, preservation, warming, thawing and the like can be carried out consistently while protecting the freezing bag.

### Brief Description of Drawings

[FIG. 1]

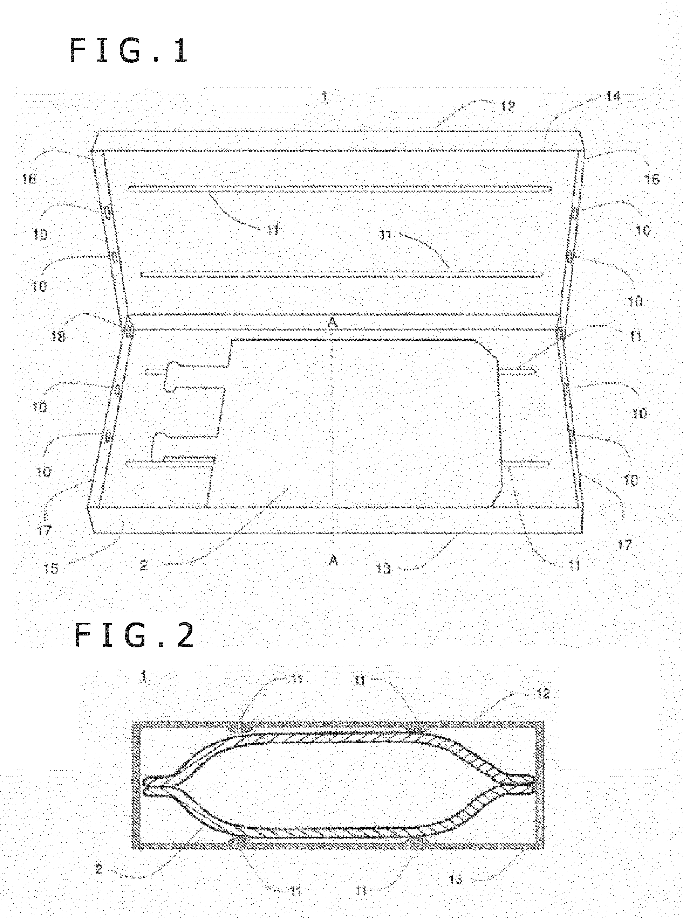

   FIG. 1 is a perspective view of a container according to a preferred embodiment of the present invention.
[FIG. 2]
   FIG. 2 is a sectional view taken along line A-A of FIG. 1.
[FIG. 3]

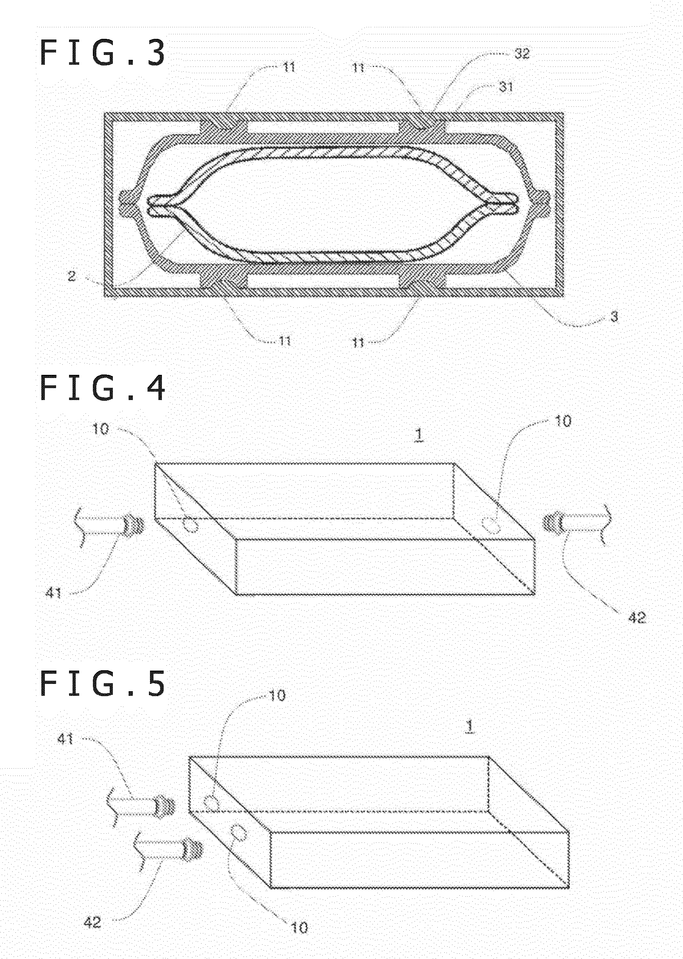

   FIG. 3 is a sectional view of a protective bag 3 for protecting a freezing bag 2.
[FIG. 4]
   FIG. 4 is a perspective view indicating an embodiment of an opening 10.
[FIG. 5]
   FIG. 5 is a perspective view indicating an embodiment of the opening 10.
[FIG. 6]

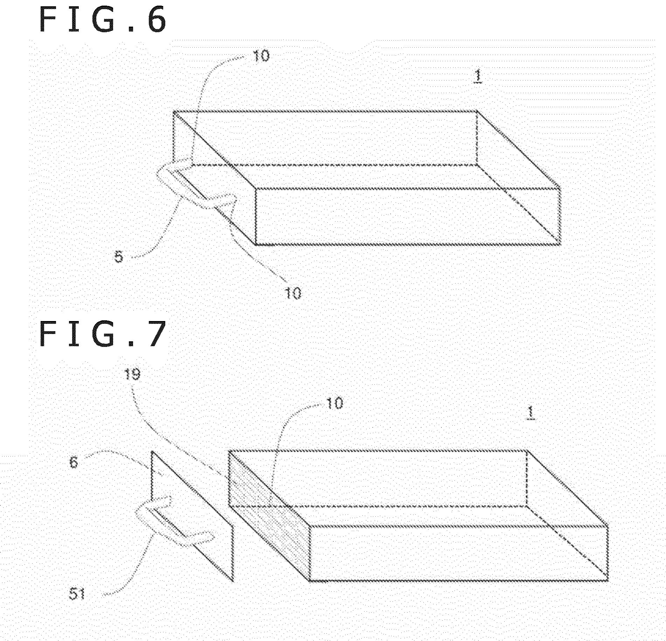

   FIG. 6 is a perspective view indicating an embodiment of the opening 10.
[FIG. 7]
   FIG. 7 is a perspective view indicating an embodiment of the opening 10.

### Modes for Carrying Out the Invention

The present invention will be described in detail below, based on preferred embodiments, while referring to the drawings. Note that the sizes of members in the drawings are appropriately exaggerated, and, therefore, they do not show actual proportions or sizes.

FIG. 1 is a perspective view of a container according to a preferred embodiment of the present invention. The container 1 has a substantially rectangular parallelepiped shape such as to be able to accommodate a freezing bag 2 therein. The container 1 has an upper portion 14 and a lower portion 15. The container 1 can be opened and closed in the state of accommodating the freezing bag 2 therein, by rotating the upper portion 14 and the lower portion 15 about a hinge 18. The container 1 has ridges 11 at an inner surface of an upper surface 12 of the upper portion 14 and at an inner surface of a lower surface 13 of the lower portion 15. Two ridges 11 are formed at each of the inner surfaces of the upper surface 12 and the lower surface 13.

The freezing bag 2 is supported by the two ridges 11 formed at the inner surface of the lower surface 13. The container 1 has openings 10 in opposed side surfaces. Two openings 10 are formed in each of the opposed side surfaces 16 of the upper portion 14 and the opposed side surfaces 17 of the lower portions 15. The openings 10 in the upper portion 14 and the openings 10 in the lower portion 15 are positioned in such a manner as to correspond to each other, and the openings 10 penetrate the side surfaces 16 of the upper portion 14 and the side surfaces 17 of the lower portion 15 in the state in which the container 1 is closed.

The material constituting the container 1 is not particularly restricted, so long as it is a material excellent in durability and thermal conductivity; preferably, the material is a rigid metallic material such as, for example, iron, stainless steel, aluminum, copper or brass. By this, the freezing bag 2 contained in the container 1 can be protected, and heat applied to the container 1 can be transferred to the freezing bag 2. The shape of the container 1 is not particularly restricted, so long as the container 1 can be hermetically sealed with the freezing bag 2 contained therein; preferably, the shape is an elongated and flat shape such that the inner surfaces of the container 1 can surround the freezing bag 2, for example. By this, the heat applied to the container 1 can be efficiently transferred to the freezing bag 2, while the freezing bag 2 is kept unmoved within the container 1. In accordance with the invention, the container 1 has a substantially rectangular parallelepiped shape such that a multiplicity of the containers 1 can be transported, stored and alignedly contained in a stable state in liquid nitrogen. The opening/closing structure of the container 1 is not specifically restricted, so long as the freezing bag 2 can be put into and taken out of the container 1.

The dimensions of the container 1 are not particularly restricted, so long as the freezing bag 2 can be contained in the container 1. For example, the length can be 50 to 500 mm, 80 to 300 mm, or 100 to 200 mm, the width can be 30 to 400 mm, 50 to 200 mm, or 80 to 150 mm, and the height can be 5 to 30 mm, 8 to 20 mm, or 10 to 15 mm.

The openings 10 are not specifically restricted, so long as fluid can move between the outside and the inside of the container 1; for example, the openings 10 may be provided in the upper surface 12, the lower surface 13, the side surfaces and the like of the container 1. The number of the openings 10 is not particularly restricted, so long as fluid can move between the outside and the inside of the container, and can be one, two, three, four, five, six, seven, eight, nine, ten or more. The size of the openings 10 is not particularly restricted, so long as durability and thermal conductivity of the container 1 can be maintained. For example, the size of the openings 10 may be set small or large according as the number of the openings 10 is large or small, whereby the durability and thermal conductivity can be maintained.

The openings 10 are provided respectively in two opposed side surfaces of the container 1. This ensures that a flow path can be formed between the opening on one side and the opening on the other side, so that fluid in the outside of the container 1 enters the inside of the container 1 via the opening on one side and moves rectilinearly toward the opening on the other side, whereby discharge of the fluid can be performed smoothly.

The ridges 11 are not specifically restricted, so long as they can form an air gap between the freezing bag 2 and the container 1; the ridges 11 are formed at an inner surface or surfaces of the lower surface 13 and/or the upper surface 12. The number of the ridges 11 is not particularly restricted, so long as the freezing bag 2 can be appropriately supported and an air gap can be formed between the freezing bag 2 and the container 1; for example, the number of the ridges 11 may be two, three, four, five, six, seven, eight, nine, ten or more. In an embodiment, two or more ridges 11 are provided at each of inner surfaces of the lower surface 13 and the upper surface 12. This ensures that the freezing bag 2 is supported appropriately, an air gap is formed both between the upper surface of the freezing bag 2 and the upper surface 12 of the container 1 and between the lower surface of the freezing bag 2 and the lower surface 13 of the container 1, and fluid flowing into the container 1 moves to both the upper surface and the lower surface of the freezing bag 2, so that the freezing bag 2 can be appropriately warmed in the case where the fluid is hot water.

The dimensions of the ridge 11 are not particularly restricted, so long as the freezing bag 2 can be supported appropriately; for example, the length of the ridge 11 can be 10 to 450 mm, 100 to 300 mm, 100 to 200 mm, 60 to 100 mm, 20 to 45 mm, or 10 to 30 mm, and the height of the ridge 11 can be 0.5 to 10 mm, or 1 to 5 mm. The shape of the ridge 11 is not specifically restricted, so long as the freezing bag 2 can be appropriately supported and an air gap can be formed between the container 1 and the freezing bag 2. Typically, the ridge 11 is a protrusion extending in a longitudinal direction and crossing the whole or part of one surface of the container 1. In accordance with the invention, where a flow path is formed between the opening on one side and the opening on the other side as aforementioned, the extending direction of the ridge 11 is set parallel to the flow path, whereby fluid can be guided along the flow path direction without hindering the flow of the fluid.

FIG. 2 is a sectional view taken along line A-A of FIG. 1. Two ridges 11 are formed at each of the inner surfaces of the upper surface 12 and the lower surface 13 of the container 1. The freezing bag 2 is supported by the two ridges 11 at the upper surface 12 of the container 1 and the two ridges 11 at the lower surface 13 of the container 1. The ridges 11 project from the inner surface of the container 1, forming an air gap between the freezing bag 2 and the inner surface of the container 1.

The material constituting the ridges 11 is not specifically restricted, so long as it is a material excellent in thermal conductivity; preferably, the material is a rigid metallic material such as, for example, iron, stainless steel, aluminum, copper or brass. By this, heat applied to the container 1 can be efficiently transferred to the freezing bag 2. The sectional shape of the ridge 11 is not specifically restricted, so long as the freezing bag 2 can be supported; for example, the sectional shape may be semicircular, arcuate, triangular, rectangular, polygonal or the like. Preferably, the sectional shape is a rounded shape. This prevents the ridges 11 from damaging the freezing bag 2 inadvertently. The ridges 11 may be molded integrally with the container 1, or may be molded as separate bodies from the container 1 and attached to the container 1.

FIG. 3 is a sectional view of a protective bag 3 for protecting the freezing bag 2. The protective bag 3 is for containing and protecting the freezing bag 2. The protective bag 3 has an opening (not indicated) for putting the freezing bag 2 in and out. The protective bag 3 has ridges 31, and the ridges 31 are formed with engaging grooves 32. The engaging grooves 32 engage with the ridges 11 of the container 1, whereby the protective bag 3 can be accurately positioned inside the container 1. The ridges 31 have a height large enough to form the engaging grooves 32 in the ridges 31, and when they are engaged with the ridges 11 of the container 1, heat applied to the container 1 can be slowly transmitted to the freezing bag 2 through the ridges 11 and the ridges 31.

The material constituting the protective bag 3 is preferably a flexible resin material excellent in flexibility or pliability. This facilitates an operation of putting the freezing bag 2 in and out. Such a resin material is not specifically restricted, and examples thereof include polyolefins such as polyethylene, polypropylene, polybutadiene, ethylene-vinyl acetate copolymer, etc., polyesters such as polyethylene terephthalate, polybutylene terephthalate, etc., flexible polyvinyl chloride, polyvinylidene chloride, silicone, polyurethane, styrene-butadiene copolymer, various thermoplastic elastomers such as polyamide elastomers, polyester elastomers, etc., and their arbitrary combinations (blend resins, polymer alloys, laminates, etc.). Polypropylene-based flexible resins are particularly preferred, from the viewpoints of sealing property, heat resistance, water resistance, flexibility, and processibility. In addition, from the viewpoints of visual observability of biological tissue in the freezing bag 2 and checkability of the position of the freezing bag 2, the material of the protective bag 3 is preferably a transparent resin material.

FIG. 4 is a perspective view indicating an embodiment of the opening 10 in the present invention. In this embodiment, one opening 10 is provided in each of two opposed side surfaces of the container 1, and a rectilinear flow path is formed between the opening on one side and the opening on the other side. A hot water pipe 41 and a drain pipe 42 can be attached to the openings 10. At the time of thawing, hot water can be delivered through the hot water pipe 41 into the container 1 to warm the freezing bag 2 in the container 1, and the hot water lowered in temperature through heat exchange can be drained through the drain pipe 42. In addition, since the hot water delivered through the hot water pipe 41 passes the air gaps formed between the freezing bag 2 and the container 1 as aforementioned, the freezing bag 2 in the container 1 can be rapidly warmed up. After the thawing of the freezing bag 2 is finished, the hot water pipe 41 and the drain pipe 42 can be detached from the openings 10. The openings 10 are each formed with, for example, a screw engagement portion, a fitting portion, an engagement portion or the like such that a pipe, for example, the hot water pipe 41, the drain pipe 42, an air feed pipe, an exhaust pipe or the like can be attached thereto.

FIG. 5 is a perspective view indicating an embodiment of the openings 10 of the present invention. In this embodiment, two openings 10 are provided in a side surface of the container 1. The hot water pipe 41 and the drain pipe 42 can be attached to the openings 10. At the time of thawing, hot water can be delivered through the hot water pipe 41 into the container 1, to warm the freezing bag 2 in the container 1, and the hot water lowered in temperature through heat exchange can be drained through the drain pipe 42.

FIG. 6 is a perspective view indicating an embodiment of the opening 10 of the present invention. In this embodiment, two openings 10 are provided in a side surface of the container 1. A handle 5 can be attached to the openings 10. This simplifies or facilitates handling of the container 1. In addition, the openings 10 can be closed by attaching the handle 5 thereto. This ensures that, for example, when the container 1 is immersed in liquid nitrogen and stored, a situation in which a foreign matter penetrates into the container 1 to damage the freezing bag 2 is avoided.

On the other hand, at the time of thawing, by detaching the handle 5 from the openings 10 to open the openings 10, then attaching the aforementioned pipe and feeding in hot water, the freezing bag 2 in the container 1 can be rapidly warmed up. In this way, the openings 10 are closed at the time of cryopreservation and the openings 10 are opened at the time of thawing, whereby a series of operations of cooling, freezing, preservation, warming, thawing and the like can be carried out efficiently and consistently. Therefore, the openings 10 of the present invention preferably have an openable and closable structure. Such an openable and closable structure can be realized by use of various known processing methods, members and assembling methods, and persons skilled in the art can appropriately determine an openable and closable structure suitable for the present invention.

FIG. 7 is a perspective view indicating an embodiment of the opening 10 in the present invention. In this embodiment, one opening 10 is provided in a side surface of the container 1. A grid-like structure body 19 is attached to the opening 10, such that the freezing bag 2 in the container 1 would not fly out inadvertently. A lid 6 can be detachably attached to the opening 10. The lid 6 is fitted with a handle 51. At the time of freezing, the lid 6 is attached to the opening 10 to close the opening 10, whereby the freezing bag 2 in the container 1 can be sealed up. In addition, at the time of thawing, the lid 6 is detached from the opening 10 to open the opening 10, whereby hot water is allowed to flow into the container 1 at a stroke.

The container of the present invention has such a configuration that thermal conductivity between the freezing bag and the container can be reduced at the time of freezing and that the area of contact between the freezing bag and hot water can be increased at the time of thawing. Therefore, excessively rapid freezing can be restrained at the time of freezing, and comparatively rapid warming can be achieved at the time of thawing. Besides, the container of the present invention enables operations of cooling, freezing, preservation, warming and thawing to be carried out consistently in a state in which the freezing bag is accommodated in and protected by the container.

While the embodiments indicated in the drawings of the present invention have been described above, the present invention is not restricted to the embodiments. The scope of the invention is defined in the appended claims.

For instance, while the container 1 has been described to be configured using a rigid metallic material in the above embodiments, this is not restrictive of the present invention. For example, the upper surface 12, the lower surface 13, the side surfaces 16 and 17, the openings 10, the ridges 11, the lid 6 and the like of the container 1 can be configured using respectively different materials. In addition, while the ridges 31 and the engaging grooves 32 have been described to be provided in the protective bag 3, they may be provided in the freezing bag 2, for example. Further, while the structure body 19 attached to the opening 10 has been described to be a grid-like body, it may be a mesh-like body. Furthermore, while the ridges 11 have been described to be longitudinally extending protrusions in the above embodiments, this is not restrictive of the present invention. For instance, the ridges 11 may each be a longitudinally extending protrusion formed by arranging a plurality of protrusions having such a shape as a conical shape, a hemispheric shape, or a rectangular shape.

### Description of Reference Symbols

- 1: Container
- 2: Freezing bag
- 3: Protective bag
- 31: Ridge
- 32: Engaging groove
- 4: Pipe
- 41: Hot water pipe
- 42: Drain pipe
- 5: Handle
- 51: Handle
- 6: Lid
- 10: Opening
- 11: Ridge
- 12: Upper surface
- 13: Lower surface
- 14: Upper portion
- 15: Lower portion
- 16: Side surface
- 17: Side surface
- 18: Hinge
- 19: Structure body

## Claims

1. A container (1) in combination with a freezing bag (2), wherein the container is thermally conductive and substantially rectangular parallelepiped-shaped, the container being adapted for containing the freezing bag (2) that in use is filled with biological tissue, and for cooling and warming the freezing bag (2), the container comprising:
at least one opening (10) formed in a side surface (16; 17) of the container (1);
at least two ridges (11; 31) formed at an inner surface of the container (1) to form an air gap between the inner surface and the freezing bag (2), wherein the opening (10) is provided in each of two opposed side surfaces (16; 17) of the container (1), to form a flow path between the opening (10) on one side and the opening (10) on the other side, and wherein the ridges (11; 31) are formed at an inner surface or surfaces of an upper surface (12) and/or a lower surface (13) of a main body of the container (1) in such a manner as to extend in parallel to a flow path direction of the flow path.

2. The container (1) according to claim 1, wherein a section of the ridge (11; 31) has an arcuate shape.

3. The container (1) according to any one of claims 1 to 2, wherein the opening (10) has such a shape as to be connectable to a pipe (4).

4. The container (1) according to any one of claims 1 to 3, further comprising a protective bag (3) for containing and protecting the freezing bag (2), wherein the protective bag (3) has ridges (11; 31) formed with engaging grooves (32) for engagement with the at least two ridges (11; 31) of the container.

5. The container (1) according to any one of claims 1 to 4, wherein the opening (10) has an openable and closable structure.

## Patentansprüche

1. Behälter (1) in Kombination mit einem Gefrierbeutel (2), wobei der Behälter wärmeleitend und im Wesentlichen rechteckig parallelepipedon-förmig ist, wobei der Behälter zur Aufnahme des Gefrierbeutels (2), der im Gebrauch mit biologischem Gewebe gefüllt ist, und zum Kühlen und Erwärmen des Gefrierbeutels (2) angepasst ist, wobei der Behälter umfasst:
mindestens eine Öffnung (10), die in einer Seitenfläche (16; 17) des Behälters (1) ausgebildet ist;
mindestens zwei Rippen (11; 31), die an einer Innenfläche des Behälters (1) ausgebildet sind, um einen Luftspalt zwischen der Innenfläche und dem Gefrierbeutel (2) zu bilden, wobei die Öffnung (10) in jeder von zwei gegenüberliegenden Seitenflächen (16; 17) des Behälters (1) vorgesehen ist, um einen Strömungsweg zwischen der Öffnung (10) auf einer Seite und der Öffnung (10) auf der anderen Seite zu bilden, und wobei die Rippen (11; 31) an einer inneren Oberfläche oder Oberflächen einer oberen Oberfläche (12) und/oder einer unteren Oberfläche (13) eines Hauptkörpers des Behälters (1) derart ausgebildet sind, dass sie sich parallel zu einer Strömungswegrichtung des Strömungsweges erstrecken.

2. Behälter (1) nach Anspruch 1, wobei ein Abschnitt der Rippe (11; 31) eine bogenförmige Form hat.

3. Behälter (1) nach einem der Ansprüche 1 bis 2, wobei die Öffnung (10) eine solche Form hat, dass sie mit einem Rohr (4) verbunden werden kann.

4. Behälter (1) nach einem der Ansprüche 1 bis 3, ferner umfassend einen Schutzbeutel (3) zur Aufnahme und zum Schutz des Gefrierbeutels (2), wobei der Schutzbeutel (3) Rippen (11; 31) aufweist, die mit Eingriffsnuten (32) zum Zusammenwirken mit den mindestens zwei Rippen (11; 31) des Behälters ausgebildet sind.

5. Behälter (1) nach einem der Ansprüche 1 bis 4, wobei die Öffnung (10) eine zu öffnende und zu schließende Struktur hat.

## Revendications

1. Conteneur (1) en association avec un sac de congélation (2), dans lequel le conteneur est thermiquement conducteur et formé de manière sensiblement parallélépipédique rectangulaire, le conteneur étant adapté afin de contenir le sac de congélation (2) qui en utilisation est rempli de tissu biologique, et de refroidir et de réchauffer le sac de congélation (2), le conteneur comprenant :
au moins une ouverture (10) formée sur une surface latérale (16 ; 17) du conteneur (1) ;
au moins deux nervures (11 ; 31) formées sur une surface interne du conteneur (1) afin de former un espace d'air entre la surface interne et le sac de congélation (2), dans lequel l'ouverture (10) est agencée sur chacune de deux surfaces latérales opposées (16 ; 17) du conteneur (1), afin de former un trajet d'écoulement entre l'ouverture (10) sur un premier côté et l'ouverture (10) sur l'autre côté, et dans lequel les nervures (11 ; 31) sont formées au niveau d'une surface interne ou des surfaces d'une surface supérieure (12) et/ou d'une surface inférieure (13) d'un corps principal du conteneur (1) d'une manière à s'étendre parallèlement à une direction de trajet d'écoulement du trajet d'écoulement.

2. Conteneur (1) selon la revendication 1, dans lequel une section transversale de la nervure (11 ; 31) présente une forme arquée.

3. Conteneur (1) selon l'une quelconque des revendications 1 à 2, dans lequel l'ouverture (10) présente une forme telle qu'elle peut être raccordée à une tuyauterie (4).

4. Conteneur (1) selon l'une quelconque des revendications 1 à 3, comprenant, en outre, un sac de protection (3) destiné à contenir et à protéger le sac de congélation (2), dans lequel le sac de protection (3) comporte des nervures (11 ; 31) comportant des rainures de couplage (32) destinées à se coupler avec les au moins deux nervures (11 ; 31) du conteneur.

5. Conteneur (1) selon l'une quelconque des revendications 1 à 4, dans lequel l'ouverture (10) présente une structure pouvant être ouverte et fermée.
